# EUROPEAN PATENT SPECIFICATION

(11) **EP 3 919 073 B1**
(45) Date of publication and mention of the grant of the patent: **15.05.2024**
(21) Application number: 21155021.5
(22) Date of filing: 03.02.2021
(51) Int. Cl.: A61K 39/00, A61P 33/00

(54) **COMPOSITION FOR PREVENTING INFESTATION AND INFECTION OF SEA LICE ON SALMON**
ZUSAMMENSETZUNG ZUR VERHINDERUNG DES BEFALLS UND DER INFEKTION MIT SEELÄUSEN AUF LACHSEN
COMPOSITION POUR PRÉVENIR L'INFESTATION ET L'INFECTION DU POU DU POISSON CHEZ LE SAUMON

(30) Priority: 05.06.2020 US 202063035050 P
(43) Date of publication of application: 08.12.2021
(73) Proprietor: Schweitzer Biotech Company Ltd., Taipei 11493 (TW)
(72) Inventor: CHEN, Charles, 11493 TAIPEI (TW); KUO, Tsun-Yung, 26341 I-Lan County (TW); Wu, Chung-Chin, I-Lan County (TW)
(74) Representative: Wittmann, Günther

(56) References cited:
- EP-B1- 2 760 888
- EP-B1- 3 405 487
- WO-A2-02/092136
- US-B2- 10 450 364
- DATABASE WPI Week 201444 Thomson Scientific, London, GB; AN 2014-F69596 XP002803577, & CN 103 554 257 A (INST HYGIENE & ENVIRONMENTAL MEDICINE MI) 5 February 2014 (2014-02-05)
- AIZENSHTEIN ELINA ET AL: "Practical aspects in the use of passive immunization as an alternative to attenuated viral vaccines", VACCINE, ELSEVIER, AMSTERDAM, NL, vol. 34, no. 22, 11 April 2016 (2016-04-11), pages 2513-2518, XP029526702, ISSN: 0264-410X, DOI: 10.1016/J.VACCINE.2016.03.051
- YI LIZHU ET AL: "Features of chicken egg yolk immunoglobulin (IgY) against the infection of red-spotted grouper nervous necrosis virus", FISH & SHELLFISH IMMUNOLOGY, ACADEMIC PRESS, LONDON, GB, vol. 80, 12 June 2018 (2018-06-12), pages 534-539, XP085429589, ISSN: 1050-4648, DOI: 10.1016/J.FSI.2018.06.024
- YAN LU ET AL: "Identification and profiling of circulating antigens by screening with the sera from schistosomiasis japonica patients", PARASITES & VECTORS, BIOMED CENTRAL LTD, LONDON UK, vol. 5, no. 1, 11 June 2012 (2012-06-11), page 115, XP021133712, ISSN: 1756-3305, DOI: 10.1186/1756-3305-5-115

## Description

### BACKGROUND OF THE INVENTION

### 1. FIELD OF THE INVENTION

The present invention relates generally to a composition for use in preventing infestation and infection of a Salmonidae by a parasite of the family *Caligidae.*

### 2. DESCRIPTION OF THE PRIOR ART

*Lepeophtheirus salmonis*, also known as salmon lice, is a species of copepod in the family *Caligidae.* Salmon lice are a type of sea lice living mostly on salmon and have been shown to reduce fish growth and appetite and cause substantial costs to salmon farmers. Salmon lice is currently one of the biggest challenges to the salmon production worldwide. This is mainly due to the increasing occurrence of resistance in salmon lice to the available agents used for treatment. Chitin synthesis inhibitors (Diflubenzuron), organophosphorous compounds (Azamethiphos), and hydrogen peroxide are the chemical agents most frequently used. An increasing problem with resistance against the most commonly used agents has pushed forward interest and research on non-medical treatment and prevention.

EP 3 405 487 B1 describes IgY antibodies for the prevention of sea lice infestation and infection of a Salmonidae, whereby said IgY antibodies are obtained by immunization of an egg-laying poultry with a Caligidae.

CN 103 554 257 A describes a new anti-schistosoma japonicum (Sjp) 40 egg yolk antibody, prepared by purifying Sjp of virus antigen immunity healthy specific pathogen-free (SPF) grade egg chicken and extracting the immunoglobulin IgY.

WO 02/092136 A2 describes immunoconjugates made of egg-yolk antibodies (IgY) from SPF hens.

EP 2 760 888 B1 describes an IgY composition for use in coeliac disease, comprising an IgY extracted from SPF hens.

Aizenshtein Elina et al., VACCINE, vol. 34, no. 22, pages 2513-2518, describes the efficacy of using specific pathogen-free (SPF) birds for antibody production taking as example the production of IgY.

US 10 450 364 B2 describes a method for preventing or treating necrotic enteritis in an avian in need thereof, comprising administering to the avian a therapeutically effective amount of a hyperimmunized egg product obtained from an egg-producing animal.

Yi Lizhu et al., FISH & SHELLFISH IMMUNOLOGY, vol. 80, pages 534-539, describes that immunization of hens that twenty 100-day-old specific pathogen free (SPF) laying hens were maintained and divided into control (10) and experimental (10) groups.

Yan Lu et al., PARASITES & VECTORS, vol. 5, no. 1, page 115 describes that 28-week-old hyline hens were immunized subcutaneously with AWA four times at an interval of 14 days with a dose of 0.5 ml (1.8 mg protein), while the AWA in PBS was used for the last immunization.

### SUMMARY OF THE INVENTION

The present invention provides a composition for use in preventing infestation and infection of a Salmonidae by a parasite of the family *Caligidae* according to claim 1.

The first aspect of the present invention relates to a composition for use in preventing infestation and infection of a Salmonidae by a parasite of the family *Caligidae*, wherein the composition comprising IgY extracted from a specific pathogen free (SPF) hen without vaccination with any antigen is administrated to the Salmonidae.

Preferably, the composition comprises egg yolk protein extract having a least 15 wt% of IgY, and wherein the egg yolk is from a specific pathogen free (SPF) hen without vaccination with any antigen, and wherein the egg yolk protein extract is obtained by delipidation and precipitation.

Preferably, the specific pathogen free (SPF) hen is not immunized with sea lice or any antigen of fish pathogen.

Preferably, the composition comprises a solution selected from the group consisting of water and phosphate buffered saline (PBS).

Preferably, the *Caligidae* is a *Lepeophtheirus.* Preferably, the *Lepeophtheirus* is *Lepeophtheirus salmonis.*

Preferably, the *Caligidae* is a *Caligus.* Preferably, the *Caligus* is *Caligus rogercresseyi.*

Preferably, the administration route is by injection. Preferably, the administration route is by injection intraperitoneally.

Preferably, at least around 10 µg of the egg yolk protein extract per gram weight of the Salmonidae is administrated to the Salmonidae.

### BRIEF DESCRIPTION OF THE DRAWINGS

**Fig. 1** shows the SDS-PAGE analysis of the egg yolk protein extract.
**Fig. 2** shows the sea lice number at 28 days post challenge in different treatment groups.

### DETAILED DESCRIPTION OF THE PREFERRED EMBODIMENT

The present invention provides a composition for use in preventing infestation and infection of a Salmonidae by a parasite of the family *Caligidae.*

The composition for use in preventing infestation and infection of a Salmonidae by a parasite of the family *Caligidae* of the present invention has demonstrated remarkable reduces in sea lice number on salmon after the salmon were injected with the composition.

Unless otherwise defined, all technical and scientific terms used herein have the same meaning as commonly understood by one of ordinary skill in the art to which this invention pertains. In the case of conflict, the present document, including definitions will control.

As used herein, the term "egg yolk" (also known as the vitellus) means the yellow internal part of a bird's egg, which is surrounded by the white, is rich in protein and fat, and nourishes the developing embryo.

As used herein, the term "egg yolk protein extract" means the protein content separated from the yellow internal part of a bird's egg.

As used herein, the term "delipidation" refers to removal of lipids or lipid groups from an egg yolk. Examples of methods for delipidation include acid water dilution, dextran sulfate precipitation, polyethylene glycol (PEG) precipitation, caprylic acid extraction, chloroform extraction, propanol-acetone extraction, and gel precipitation.

As used herein, the term "precipitation" refers to the process of conversion of soluble proteins in an egg yolk into a solid from a solution of delipidated egg yolk by converting the soluble proteins into an insoluble form or a super-saturated solution. Examples of methods for precipitation include ammonium sulfate precipitation, filtration, ion exchange chromatography, thiophilic interaction chromatography, and immunoaffinity chromatography.

As used herein, the term "hen" means a female bird that can produce eggs. Examples of birds used in the present invention includes chicken, turkey, ostrich, goose, duck, pigeon, parrot, and fowl.

As used herein, the term "specific-pathogen-free (SPF)" is used for laboratory animals that are guaranteed free of particular pathogens. As used herein, the specific pathogen includes avian adenovirus group III, avian encephalomyelitis virus (AEV), avian influenza-Type A virus (AIV-A), chicken anaemia virus (CAV), *Haemophilus paragallinarum*, avian infectious bronchitis virus (IBV), infectious bursal disease virus (IBDV), infectious Laryngotracheitis virus (ILTV), avian leukosis virus (ALV)-A, B, and J, Marek's disease virus (MDV), *Mycoplasma gallisepticum*, *Mycoplasma synoviae*, Newcastle disease virus (NDV), *Salmonella pullorum*, avian metapneumovirus (AMPV), avian reovirus (ARV), inclusion body hepatis virus (IBHV), fowl pox virus (FPV), avian nephritis virus (ANV), avian reticulonedotheliosis virus (AREV), *Salmonella enteriditis* (SE), avian lymphoid leukosis groups A and B, chicken infectious anaemia virus (CIA), *Chlamydia psittaci*, egg drop syndrome virus (EDSV), reticuloendotheliosis virus (REV), Gallid herpesvirus 1 (GaHV-1), *Avibacterium paragallinarum*,

As used herein, the term "fish pathogen" refers to such as piscine reovirus (PRV), infectious pancreatic necrosis virus (IPNV), infectious salmon anemia virus (ISAV), infectious pancreatic necrosis virus (IPNV), salmonid alphavirus (SAV), piscine *myocarditis virus (PMCV), salmon gill poxvirus (SGPV), tilapia Lake Virus (TiLV), Koi herpesvirus (KHV), Nervous necrosis virus (NNV), Lymphocystis virus, Aeromonas salmonicida ssp., Renibacterium salmoninarum, Yersinia ruckeri, Piscirickettsia salmonis, Candidatus Branchiomonas cysticola (CBc), Desmozoon lepeophtherii (Des), Neoparamoeba perurans, Photobacterium damselae subsp. Piscicida, vibrio, cnidarian parasites, flatworms, and sea lice.*

*As used herein, the singular forms "a ", "an "*, *and "the " include plural referents unless the context clearly dictates otherwise. Thus*, for example, reference to "a component" includes a plurality of such components and equivalents thereof known to those skilled in the art.

As used herein, "around", "about" or "approximately" shall generally mean within 20 percent, preferably within 10 percent, and more preferably within 5 percent of a given value or range. Numerical quantities given herein are approximate, meaning that the term "around", "about" or "approximately" can be inferred if not expressly stated.

The present invention is described in more detail in the following illustrative examples. Although the examples may represent only selected embodiments of the invention, it should be understood that the following examples are illustrative.

### EXAMPLES

### Example 1 Preparation of Egg Yolk Protein Extract

Egg yolk obtained from specific pathogen free (SPF) hens without vaccination with any antigen were collected. Protein extraction of the egg yolk was performed as follows. The yolk was mixed with five (5) volumes of delipidation solution (water acidified with HCl, pH 5.0-5.2; or 1.2x containing 0.06% κ-carrageenan, 0.18% low-methoxyl pectin, and 10 µM CaCl₂) until homogeneous. The mixture was then incubated at 4°C for 2 hours and centrifuged for 20 minutes at 11,000 xg at 4°C, and the precipitated lipoprotein and lipids was discarded. Soluble protein in the resultant delipidated supernatant was precipitated using (NH₄)₂SO₄ to a final concentration of 30-60% and then incubated for 1 hour at 4°C and centrifuged for 20 minutes at 11,000 xg at 4°C. The pellet was resuspended with three (3) volume of phosphate buffered saline (PBS) and stored at 2°C to 8°C until used.

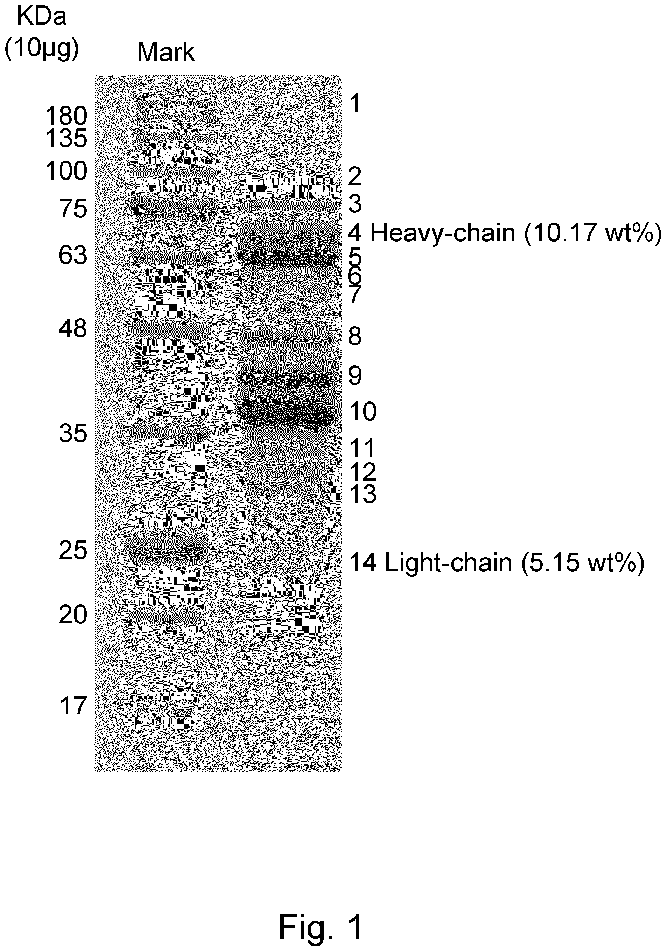

Lipids were removed from the egg yolk protein extract, and the protein content of the yolk extract was measured by Pierce^{™} BCA Protein Assay Kit (Thermo Fisher Scientific, Waltham, MA, U.S.A.). Ten (10) micrograms of total protein of the yolk extract was run on a 12% sodium dodecyl sulphate-polyacrylamide gel electrophoresis (SDS-PAGE) to analyze the content of the yolk extract. As shown in **Figure 1****,** the result indicates that the yolk extract has around 15 wt% of IgY (around 10.17 wt% heavy chain of IgY and around 5.15 wt%). The obtained egg yolk protein extract was stored at 2°C to 8°C for further use.

### Example 2 Efficacy of IgY Against Infection of Sea Lice on Salmon

Atlantic salmon (*Salmo salar* L.) of 300-350g size were kept at 32%o seawater (8°C). The fish were kept in three different tanks. Fish in different tanks were treated as follows.
Tank 1: Forty-five (45) fish were injected intraperitoneally with PBS. (Control group)
Tank 2: Fifteen (15) fish were injected intraperitoneally with 10 µg egg yolk protein extract produced in Example 1/g fish weight and marked by fin-clipping. In addition, 15 fish were injected intraperitoneally with 150 µg egg yolk protein extract produced in Example 1/g fish weight.
Tank 3: Fifteen (15) fish were injected intraperitoneally with 10 µg egg yolk protein extract produced in Example 1/g fish weight and marked by fin-clipping. In addition, 15 fish were injected intraperitoneally with 150 µg egg yolk protein extract produced in Example 1/g fish weight.

The fish in Tanks 1-3 were injected intraperitoneally using a 24G needle, 7 days prior to challenge with sea lice copepodids.

Twenty (20) nauplii of *Lepeophtheirus salmonis*, per fish were added to a tank containing 200 L of seawater (3.2%). The water flow was turned off during the challenge with the sea lice, and the water was oxygenated through an external source and monitoring continuously using a standard oxygen probe. The concentration of oxygen was not allowed to fall below 8%. The copepodids were allowed to attach to the fish for 30 minutes after which the water flow was resumed and oxygenation ceased.

Counting of sea lice was done at 28 days post challenge. Percentage of effect of the treatment was calculated with the following equation. % Effect = (Sea lice number of Control - sea lice number of Treatment)/(Sea lice number of Control) x 100%

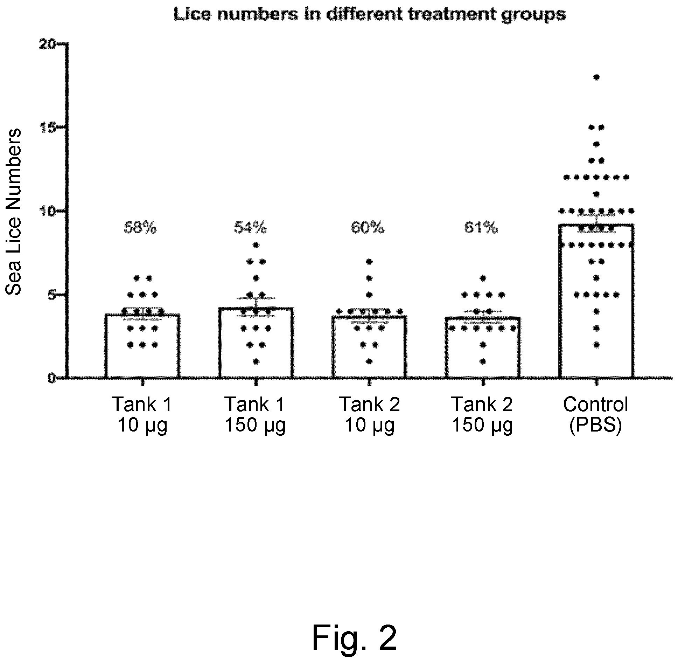

As shown in **Figure 2** and **Table 1,** fish injected with PBS (Tank 1) had an average of 9.26 sea lice per fish. Fish injected with 10 µg/g fish weight of yolk extract produced in Example 1 in Tank 2 had an average of 3.87 sea lice per fish, and fish injected with 150 µg/g fish weight of yolk extract produced in Example 1 in Tank 2 had an average of 4.27 sea lice per fish. Fish injected with 10 µg/g fish weight of yolk extract produced in Example 1 in Tank 3 had an average of 3.73 sea lice per fish, and fish injected with 150 µg/g fish weight of yolk extract produced in Example 1 in Tank 3 had an average of 3.67 sea lice per fish. Putting the data of same treatment together, fish injected with 10 µg/g fish weight of yolk extract produced in Example 1 had an average 3.80 sea lice per fish and 58.96% effect of protecting salmon from sea lice infection. Similarly, fish injected with 150 µg/g fish weight of yolk extract produced in Example 1 had an average 3.97 sea lice per fish and 57.13% effect of protecting salmon from sea lice infection.

**Table 1 Results of Challenge with Sea Lice**

| Treatment | Average See Lice/Fish | % reduction | Combined Average See Lice/Fish | Combined % reduction |
|---|---|---|---|---|
| Injection with PBS (Tank 1) (Control) | 9.26 | 0% | - | - |
| Injection with 10 µg/g fish weight (Tank 2) | 3.87 | 58.21% | 3.80 | 58.96% |
| Injection with 10 µg/g fish weight (Tank 3) | 3.73 | 59.72% | | |
| Injection with 150 µg/g fish weight (Tank 2) | 4.27 | 53.89% | 3.97 | 57.13% |
| Injection with 150 µg/g fish weight (Tank 2) | 3.67 | 60.38% | | |

The results of sea lice challenge indicate that administration of the composition of the present invention prevents and inhibits infestation and/or attachment of sea lice to the fish challenged with sea lice. Therefore, the composition of the present invention can be used as a prophylactic and/or curative measure against sea lice infection.

## Claims

1. A composition for use in preventing infestation and infection of a Salmonidae by a parasite of the family *Caligidae,* wherein the composition comprises IgY extracted from a specific pathogen free (SPF) hen without vaccination with any antigen and the composition is administrated to the Salmonidae.

2. The composition for use according to claim 1, wherein the composition comprises egg yolk protein extract having at least 15 wt% of IgY, and wherein the egg yolk is from a specific pathogen free (SPF) hen without vaccination with any antigen, and wherein the egg yolk protein extract is obtained by delipidation and precipitation.

3. The composition for use according to claim 1 or 2, wherein the specific pathogen free (SPF) hen is not immunized with sea lice or any antigen of fish pathogen.

4. The composition for use according to any of the claims 1 to 3, wherein the *Caligidae* is a *Lepeophtheirus* or a *Caligus.*

5. The composition for use according to claim 4, wherein the *Lepeophtheirus* is *Lepeophtheirus salmonis.*

6. The composition for use according to claim 4, wherein the *Caligus* is *Caligus rogercresseyi.*

7. The composition for use according to any of the claims 1 to 6, wherein the composition comprises a solution selected from the group consisting of water and phosphate buffered saline (PBS).

8. The composition for use according to any of the claims 1 to 7, wherein the administration route is by injection.

9. The composition for use according to any of the claims 2 to 8, wherein at least around 10 µg of the egg yolk protein extract per gram weight of the Salmonidae is administrated to the Salmonidae.

## Patentansprüche

1. Zusammensetzung zur Verwendung bei der Verhinderung des Befalls und der Infektion von Salmoniden durch einen Parasiten aus der Familie der Caligidae, wobei die Zusammensetzung IgY umfasst, das aus einem spezifisch pathogenfreien (SPF) Huhn extrahiert wurde, ohne dass es mit irgendeinem Antigen geimpft wurde, und die Zusammensetzung den Salmoniden verabreicht wird.

2. Zusammensetzung zur Verwendung gemäß Anspruch 1, wobei die Zusammensetzung einen Eigelbproteinextrakt mit mindestens 15 Gew.-% IgY umfasst, und wobei das Eigelb von einem spezifisch pathogenfreien (SPF) Huhn ohne Impfung mit irgendeinem Antigen stammt, und wobei der Eigelbproteinextrakt durch Delipidierung und Ausfällung erhalten wird.

3. Zusammensetzung zur Verwendung gemäß Anspruch 1 oder 2, wobei das spezifisch pathogenfreie (SPF) Huhn nicht mit Seeläusen oder irgendeinem Antigen eines Fischpathogens immunisiert ist.

4. Zusammensetzung zur Verwendung gemäß einem der Ansprüche 1 bis 3, wobei die Caligidae ein Lepeophtheirus oder ein Caligus ist.

5. Zusammensetzung zur Verwendung gemäß Anspruch 4, wobei der Lepeophtheirus Lepeophtheirus salmonis ist.

6. Zusammensetzung zur Verwendung nach Anspruch 4, wobei der Caligus Caligus rogercresseyi ist.

7. Zusammensetzung zur Verwendung nach einem der Ansprüche 1 bis 6, wobei die Zusammensetzung eine Lösung umfasst, die aus der Gruppe ausgewählt ist, die aus Wasser und phosphatgepufferter Kochsalzlösung (PBS) besteht.

8. Zusammensetzung zur Verwendung nach einem der Ansprüche 1 bis 7, wobei der Verabreichungsweg durch Injektion erfolgt.

9. Zusammensetzung zur Verwendung nach einem der Ansprüche 2 bis 8, wobei den Salmoniden zumindest etwa 10 µg des Eigelbproteinextrakts pro Gramm Gewicht der Salmoniden verabreicht werden.

## Revendications

1. Composition pour une utilisation au niveau de la prévention de l'infestation et de l'infection d'un salmonidé (famille Salmonidae) par un parasite de la famille *Caligidae*, dans laquelle la composition comprend des IgY extraits à partir d'une poule exempte de pathogènes spécifiques (SPF) sans vaccination avec un quelconque antigène et la composition est administrée au salmonidé (famille Salmonidae).

2. Composition pour une utilisation selon la revendication 1, dans laquelle la composition comprend de l'extrait de protéine de jaune d'oeuf comportant au moins 15 % en poids d'lgY, et dans laquelle le jaune d'oeuf provient d'une poule exempte de pathogènes spécifiques (SPF) sans vaccination avec un quelconque antigène, et dans laquelle l'extrait de protéine de jaune d'oeuf est obtenu par délipidation et précipitation.

3. Composition pour une utilisation selon la revendication 1 ou 2, dans laquelle la poule exempte de pathogènes spécifiques (SPF) n'est pas immunisée avec le pou de mer ou un quelconque antigène de pathogène du poisson.

4. Composition pour une utilisation selon l'une quelconque des revendications 1 à 3, dans laquelle le *Caligidae* est un *Lepeophtheirus* ou un *Caligus.*

5. Composition pour une utilisation selon la revendication 4, dans laquelle le *Lepeophtheirus* est le *Lepeophtheirus salmonis.*

6. Composition pour une utilisation selon la revendication 4, dans laquelle le *Caligus* est le *Caligus rogercresseyi.*

7. Composition pour une utilisation selon l'une quelconque des revendications 1 à 6, dans laquelle la composition comprend une solution sélectionnée parmi le groupe constitué par l'eau et une solution saline tamponnée au phosphate (PBS).

8. Composition pour une utilisation selon l'une quelconque des revendications 1 à 7, dans laquelle le mode d'administration est par injection.

9. Composition pour une utilisation selon l'une quelconque des revendications 2 à 8, dans laquelle au moins environ 10 µg de l'extrait de protéine de jaune d'oeuf par poids en grammes du salmonidé (famille Salmonidae) sont administrés au salmonidé (famille Salmonidae).
